# EUROPÄISCHE PATENTSCHRIFT

(11) **EP 1 761 309 B1**
(45) Veröffentlichungstag und Bekanntmachung des Hinweises auf die Patenterteilung: **09.09.2009**
(21) Anmeldenummer: 05763189.7
(22) Anmeldetag: 09.06.2005
(51) Int. Cl.: A61N 5/10

(54) **BESTRAHLUNGSEINRICHTUNG**
IRRADIATION APPARATUS
DISPOSITIF D'APPLICATION DE RAYONNEMENT

(30) Priorität: 09.06.2004 DE 102004029026
(43) Veröffentlichungstag der Anmeldung: 14.03.2007
(73) Patentinhaber: Rhön-Klinikum AG, 97616 Bad Neustadt/Saale (DE)
(72) Erfinder: MÜNCH, Eugen, Rhön-Klinikum AG, 97616 Bad Neustadt/Saale (DE)
(74) Vertreter: Gleiss & Grosse
(86) Internationale Anmeldenummer: PCT/EP2005/006209
(87) Internationale Veröffentlichungsnummer: WO 2005/120640

(56) Entgegenhaltungen:
- EP-A- 1 430 932
- WO-A-01/66187
- US-A- 5 190 516
- DATABASE INSPEC [Online] THE INSTITUTION OF ELECTRICAL ENGINEERS, STEVENAGE, GB; December 2001 (2001-12), BRAHME A ET AL: "Design of a centre for biologically optimised light ion therapy in Stockholm" Database accession no. 7186670 & NUCLEAR INSTRUMENTS & METHODS IN PHYSICS RESEARCH, SECTION B (BEAM INTERACTIONS WITH MATERIALS AND ATOMS) ELSEVIER NETHERLANDS, vol. 184, no. 4, pages 569-588, ISSN: 0168-583X

## Beschreibung

Die Erfindung betrifft eine Bestrahlungseinrichtung für die Protonen- und/oder Ionenstrahltherapie gemäß Oberbegriff des Anspruchs 1. Derartige, auch als Ionentherapiebeschleuniger bezeichnete, Bestrahlungseinrichtungen, weisen im Allgemeinen eine Beschleunigeranlage zur Erzeugung und Beschleunigung der Ionen auf die gewünschte Energie, also eine Strahlungsquelle, sowie Patientenbehandlungsräume und Aufenthaltsräume für das Bedienerpersonal auf.

Ionenstrahlen, das heißt Protonen und Schwerionen, werden zur Strahlentherapie von Tumorgeweben eingesetzt. In den USA und in Europa werden lonentherapieanlagen für den klinischen Routinebetrieb geplant und sind bereits im Aufbau oder kurz vor der Fertigstellung. In hochenergetischen Beschleunigeranlagen entsteht an verschieden Stellen Sekundärstrahlung, die durch Abbremsprozesse der Ionen mit Materie erzeugt werden. Insbesondere wird bei der Bestrahlung von Patienten durch die Wechselwirkung der Ionen mit dem Gewebe des Patienten Sekundärstrahlung, hauptsächlich Neutronenstrahlung, erzeugt. Diese Neutronenstrahlung weist eine starke Richtungsabhängigkeit auf, das heißt, sie ist vom Erzeugungsort aus gesehen strahlabwärts gerichtet und verläuft in einem sogenannten Strahlungskegel, der einen Öffnungswinkel von ungefähr ± 20° aufweist.

Da sich während der Patientenbestrahlung mehrere Personen gleichzeitig in dem Gebäude und in der Umgebung des Beschleunigers sowie der Behandlungsräume aufhalten, ist die Abschirmung der entstehenden Strahlung von großer Bedeutung. In einem klinischen Routinebetrieb werden immer mehrere Behandlungsplätze für Patientenbestrahlung in unmittelbarer Nähe zueinander vorzufinden sein, sodass auch die Patientenbehandlungsplätze untereinander abgeschirmt sein müssen. In der WO 2004/013865 ist ein abgeschirmter Raum, der Patientenbehandlungs- oder Therapieraum genannt wird, für die Ionentherapie für Neutronen bis in den Energiebereich GeV beschrieben. Der Zugang zu diesem Raum weist mindestens zwei in Längsrichtung zueinander versetzte Abschirmungselemente auf, die von entgegengesetzten Wänden des Zugangs entspringen und jeweils mehr als die Hälfte der lichten Weite des Zugangs abdecken. Hierbei ist nachteilig, dass der Zugang sich strahlabwärts, das heißt in dem vorwärts gerichteten Neutronenkegel, also der Hauptstrahlungsintensität, befindet.

Aufgabe der Erfindung ist es daher, eine Bestrahlungseinrichtung mit Behandlungsräumen, in die ein Therapiestrahl eingeleitet wird, bezüglich der Abschirmung der Behandlungsräume derart auszugestalten, dass der Zugang besser gegen Neutronenstrahlung geschützt ist.

Zur Lösung dieser Aufgabe wird eine Bestrahlungseinrichtung für die Protonen- und/oder Ionenstrahltherapie vorgeschlagen, die eine Strahlungsquelle und mindestens zwei eine Abschirmung und einen Behandlungsort aufweisende Behandlungsräume umfasst, wobei ein Therapiestrahl in die Behandlungsräume eingeleitet wird. Die Bestrahlungseinrichtung zeichnet sich dadurch aus, dass der Zugang, in Richtung des Therapiestrahls gesehen, strahlaufwärts zum Behandlungsort angeordnet ist und in jeden der Behandlungsräume ein separater Therapiestrahl eingeleitet wird. Da die Hauptintensität der am Behandlungsort, also im Patienten, erzeugten Neutronenstrahlung strahlabwärts gerichtet ist, ist der Bereich vor dem Patienten, also strahlaufwärts gesehen, nur durch eine sehr geringe Neutronenstrahlung belastet. In jeden der separaten Behandlungsräume wird ein separater Therapiestrahl geleitet. Die den Behandlungsräumen zugeordneten Therapiestrahlen sind unabhängig voneinander steuerbar. Während in einem Behandlungsraum ein Patient bestrahlt wird, können - unabhängig davon - in einem anderen Behandlungsraum ohne Gefährdung andere Arbeiten durchgeführt werden, ohne dass die sich darin befindenden Personen durch Neutronenstrahlung belastet werden. Ferner ist ein rascher Wechsel von Patienten möglich.

Bevorzugt wird ein Ausführungsbeispiel, bei dem mindestens ein Behandlungsraum symmetrisch oder asymmetrisch ausgelegt ist. Die unterschiedliche Auslegung der Behandlungsräume erlaubt eine individuelle Abstimmung auf die im Behandlungsraum benötigten zusätzlichen Aufbauten, beispielsweise medizinische Diagnosegeräte.

Bei einem weiteren bevorzugten Ausführungsbeispiel weisen die Behandlungsräume, in Richtung des Therapiestrahls gesehen, eine geschlossene Abschirmung auf. Eine geschlossene Abschirmung, das heißt eine Abschirmung, die nicht beispielsweise durch Zugangstüren unterbrochen ist, gewährleistet eine effektivere Abschirmung der Neutronenstrahlung.

Bei einem weiteren bevorzugten Ausführungsbeispiel weist der Zugang ein Labyrinth auf. Ein labyrinthartig ausgestalteter Zugang hat den Vorteil, dass an den Wänden dieses Labyrinths die Neutronenstrahlung durch Reflektion zusätzlich zu der Abschirmung abgeschwächt wird.

Bei einem weiteren bevorzugten Ausführungsbeispiel ist das Labyrinth durch separate Wände realisiert. Hierbei ist eine große Flexibilität in der architektonischen Auslegung der Behandlungsräume gewährleistet.

Bei einem weiteren bevorzugten Ausführungsbeispiel ist das Labyrinth durch Wände benachbarter Behandlungsräume realisiert. Dies erlaubt eine besonders dichte, raumsparende Anordnung mehrerer Behandlungsräume nebeneinander.

Bei einem weiteren Ausführungsbeispiel verläuft der Zugang teilweise entlang einer als Abschirmwand ausgelegten Außenwand des zugehörigen Behandlungsraumes. Die Abschirmwand dient hierbei gleichzeitig als Begrenzungswand für den Zugang und erlaubt einen kostengünstigen Aufbau der Behandlungsräume.

Bei einem weiteren bevorzugten Ausführungsbeispiel wird der Therapiestrahl aus einem aus der Strahlungsquelle austretenden Hochenergiestrahl ausgelenkt. Der Hochenergiestrahl umfasst die für die Therapie verwendeten Ionen mit der zugehörigen Energie. Dies ist beispielsweise ein Kohlenstoffstrahl mit 400 MeV/amu. Als Strahlungsquelle ist hier der Teil des gesamten Beschleunigers gemeint, der die erforderliche Energie und Ionensorte für den Therapiestrahl bereitstellt. Dies kann beispielsweise bei einer Anordnung bestehend aus Ionenquellen, Linearbeschleuniger und Synchrotron bestehenden Beschleunigeranlage das Synchrotron sein oder bei einer Beschleunigeranlage, die ein Zyklotron aufweist, das Zyklotron. Auf jeden Fall ist festzuhalten, dass mit dem Begriff Strahlungsquelle hier der Beschleunigerteil angesprochen wird, aus dem die Ionen mit der gewünschten Ionensorte und der gewünschten Ionenenergie austreten. Die Anordnung, bei der der Hochenergiestrahl aus der Strahlungsquelle ausgelenkt wird und von diesem Therapiestrahlen ausgelenkt werden, ist deswegen vorteilhaft, weil von dem Hochenergiestrahl mehrere Therapiestrahlen ausgelenkt werden können und somit mehrere Behandlungsräume für Patienten, in die jeweils ein Therapiestrahl eingeleitet wird, entlang des Hochenergiestrahls angeordnet werden können.

Bei einem weiteren bevorzugten Ausführungsbeispiel erfolgt die Auslenkung des Therapiestrahls unter einem Winkel α. Bei einem Winkel α = 0° wird der Therapiestrahl in Geradeausrichtung aus dem Hochenergiestrahl gewonnen. Bei Winkeln, die zwischen 0° und 90° liegen, können mehrere Therapiestrahlen, die jeweils parallel zueinander liegen, entweder an einer Seite des Hochenergiestrahls oder an beiden Seiten des Hochenergiestrahls ausgelenkt werden. Hierbei ist eine optimale Ausnutzung der Räumlichkeiten gewährleistet.

Bei einem bevorzugten Ausführungsbeispiel liegen die Behandlungsräume in Reihe - vorzugsweise parallel - nebeneinander. Die Anordnung der Behandlungsräume in Reihen erlaubt möglichst kurze Zugänge zu den einzelnen Behandlungsräumen und die Verwendung der Abschirmungen der Behandlungsräume als Wände für die einzelnen Zugänge. Es ist möglich, eine Reihe von Behandlungsräumen vorzusehen, oder zwei Reihen von Behandlungsräumen, die dann jeweils auf beiden Seiten des Hochenergiestrahls angeordnet sind.

Bei einem weiteren bevorzugten Ausführungsbeispiel ist vorgesehen, die Behandlungsräume in zwei Reihen in einer Ebene - vorzugsweise parallel - nebeneinander anzuordnen.

Bei einem weiteren Ausführungsbeispiel ist es vorgesehen, die Behandlungsräume in unterschiedlichen Ebenen anzuordnen. Mit Ebenen sind hier verschiedene Stockwerke der lonenstrahltherapieanlage gemeint. Hierbei ist vorteilhaft, dass die Behandlungsräume nicht gleichartig ausgelegt sein müssen.

Bei einem weiteren Ausführungsbeispiel ist vorgesehen, dass die Behandlungsräume sternförmig um die Strahlungsquelle angeordnet sind. Hierbei wird ermöglicht, dass an verschiedenen Stellen der Strahlungsquelle ein Hochenergiestrahl ausgelenkt wird. Dieser kann dann auf zwei verschiedene Weisen genutzt werden. Zum einen ist es möglich, aus diesem Hochenergiestrahl verschiedene Therapiestrahlen unter einem Winkel α auszuleiten, sodass die oben diskutierte Anordnung von Behandlungsräumen, die in Reihe - vorzugsweise parallel - nebeneinander liegen. Zum anderen ist es möglich, einen Hochenergiestrahl auszulenken und diesem einen separaten Behandlungsraum zuzuordnen. Dies kann beispielsweise notwendig sein, wenn ein Behandlungsraum besonders groß ausgestaltet werden muss, um hier große Geräte unterzubringen. Somit ist durch die sternförmige Anordnung der Behandlungsräume um die Strahlungsquelle eine größtmögliche Flexibilität sowohl in der Anzahl, als auch in der Ausgestaltung von Behandlungsräumen gewährleistet.

Bei einem weiteren bevorzugten Ausführungsbeispiel ist vorgesehen, dass die Abschirmung des Behandlungsraumes Metall und/oder ein wasserstoffhaltiges Material und/oder Graphit aufweist. Hierbei ist vorteilhaft, dass Abschirmungen, die durch verschiedene Mechanismen eine Abschwächung der Neutronenstrahlung bewirken, kombiniert werden können.

Bei einem bevorzugten Ausführungsbeispiel ist vorgesehen, dass das wasserstoffhaltige Material Paraffin und/oder gebundenes Wasser aufweist. Paraffin ist besonders vorteilhaft, weil aus diesem sehr einfach Blätter geschnitten werden können, die in verschiedenen Größen anfertigbar sind. Gebundenes Wasser liegt in verschiedenen Formen vor, beispielsweise in Beton oder in Gips, wo das Wasser als sogenanntes Kristallwasser auftritt. Auch hierbei ist eine größtmögliche Flexibilität in der Ausgestaltung und dem Aufbau der Abschirmung gewährleistet.

Bei einem weiteren bevorzugten Ausführungsbeispiel ist vorgesehen, dass das Metall Eisen und/oder Blei umfasst. Eisen ist ein bekannter Werkstoff, der einfach in Beton einarbeitbar ist. Auf diese Weise lassen sich einfach und auf kostengünstige Weise Wände, die sowohl stabil sind als auch Abschirmeigenschaften aufweisen, herstellen. Blei kann in einfacher Weise zu dünnen Folien ausgewalzt werden, die dann in eine Wand, die beispielsweise aus Beton oder Gips besteht, leicht eingefügt werden können und eine gute Abschirmwirkung zeigen.

Bei einem weiteren bevorzugten Ausführungsbeispiel ist vorgesehen, dass die Abschirmung ein Neutronen absorbierendes Material aufweist. Dieses kann vorzugsweise Bor und/oder Bor-Paraffin und/oder Cadmium und/oder Gadolinium sein. Diese Materialien sind dafür bekannt, dass sie eine große neutronenabsorbierende Wirkung haben. Somit ist eine zusätzliche Abschirmung der Neutronen ermöglicht. Alle diese Materialien sind einfach in die Grundstruktur von Wänden aus Beton einfügbar.

Bei einem weiteren bevorzugten Ausführungsbeispiel sind die Wände des Behandlungsraumes etwa G-förmig angeordnet. Durch diese Anordnung ist ein Labyrinth realisierbar, in dem die Neutronen durch Streuung an den Wänden zusätzlich abgeschwächt werden.

Ein weiteres bevorzugtes Ausführungsbeispiel zeichnet sich dadurch aus, dass die Wände des Behandlungsraumes spiralförmig um den Behandlungsort angeordnet sind. Hierdurch kann eine mehrlagige Abschirmung realisiert werden, wobei sich der Zugang zwischen den Abschirmwänden befindet. Aus bautechnischen Gründen kann es notwendig sein, die Abschirmwände dünner auszugestalten, dafür aber zwei Wände mit zwischenliegenden Zugängen vorzusehen.

Ein weiteres bevorzugtes Ausführungsbeispiel zeichnet sich dadurch aus, dass der Behandlungsraum eine als Notzugang und/oder Servicezugang dienende Tür aufweist. Hierbei weist die Tür Material der Abschirmung auf. Dies bedeutet, dass die Tür in die Abschirmung integriert ist und wie die Abschirmung bezüglich der Neutronenstrahlung wirkt. Soll beispielsweise in dem Behandlungsraum ein größeres Gerät eingebracht werden, kann es notwendig sein, eine Tür zu haben, die größere Maße als die Standardzugänge aufweist. Die Standardzugänge weisen typischerweise eine Breite von zwei Metern auf, sodass es vorteilhaft ist, einen Zugang mit einer Tür vorzusehen, die eine Breite von mehr als zwei Metern aufweist.

Bei einem weiteren Ausführungsbeispiel ist vorgesehen, dass die Tür versenkbar ist. Hierbei ist vorteilhaft, dass die Tür bei Arbeiten im Behandlungsraum in geöffnetem Zustand nicht im Wege steht.

Weitere Ausgestaltungen ergeben sich aus den Unteransprüchen.

Die Erfindung wird im Folgenden anhand der Zeichnung näher erläutert. Es zeigen:
- Figur 1: eine Bestrahlungseinrichtung mit einer Strahlungsquelle und Behandlungsräumen;
- Figur 2: eine Strahlungsquelle, um die sternförmig Behandlungsräume angeordnet sind;
- Figur 3: eine Prinzipskizze eines Behandlungsraumes, der symmetrisch gestaltet ist;
- Figur 4: eine Prinzipskizze eines Behandlungsraumes, der asymmetrisch gestaltet ist;
- Figur 5: eine Anordnung von mehreren Behandlungsräumen, nebeneinander unter einem Winkel von 45° zum Hochenergiestrahl.

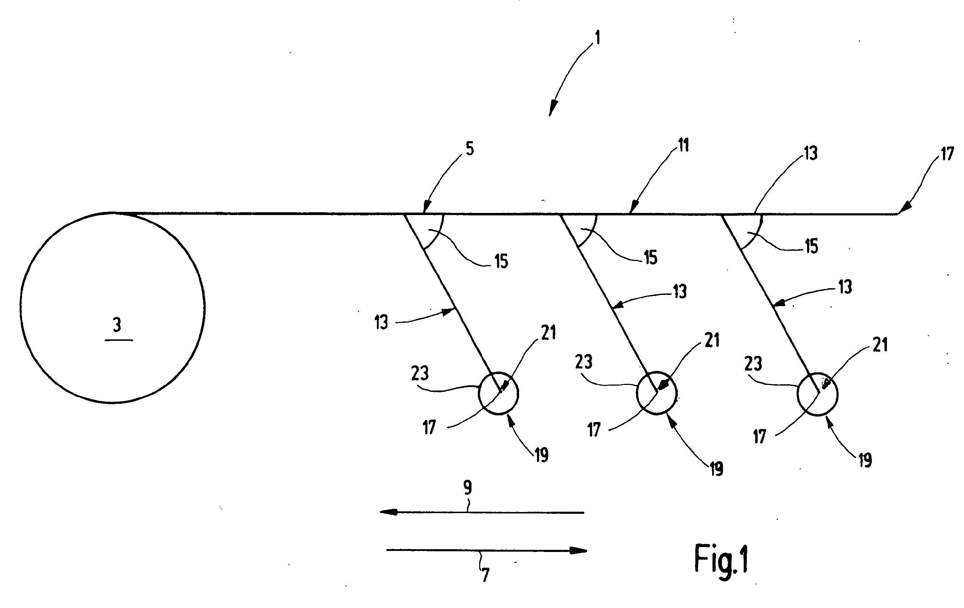

Figur 1 zeigt eine Bestrahlungseinrichtung 1, die eine Strahlungsquelle 3 und einen Hochenergiestrahl 5 umfasst. Die Strahlungsquelle 3 ist die Quelle für die zur Bestrahlung eines Patienten verwendeten Ionen, die die für die Bestrahlung notwendige Energie aufweisen. Die Strahlungsquelle 3 kann ein ein Synchrotron umfassender Therapiebeschleuniger oder ein ein Zyklotron umfassender Therapiebeschleuniger sein. Es ist auch möglich, andere Therapiebeschleuniger zu verwenden. Entscheidend ist an dieser Stelle lediglich, dass Ionen, die aus der Strahlungsquelle 3 austreten, die Ionensorte mit der Ionenenergie aufweisen, die für die Tumortherapie eingesetzt werden kann. Der auch als Ionenstrahl bezeichnete Hochenergiestrahl 5 verläuft in der mit dem Pfeil 7 bezeichneten Richtung von der Strahlungsquelle zu dem zu behandelnden Patienten nach rechts weg. Die mit dem Pfeil 7 bezeichnete Richtung wird im Folgenden als strahlabwärts bezeichnet. Die mit dem Pfeil 9 gekennzeichnete, entgegengesetzte Richtung wird im Folgenden als strahlaufwärts bezeichnet.

Der Hochenergiestrahl 5 wird in der Hauptstrahlführung 11 geführt. Von der Hauptstrahlführung 11 wird aus dem Hochenergiestrahl 5 ein Therapiestrahl 13 ausgelenkt. In diesem Ausführungsbeispiel werden mehrere Therapiestrahlen 13 unter dem mit 15 bezeichneten Winkel a aus dem Hochenergiestrahl 5 ausgelenkt. Dargestellt sind vier Therapiestrahlen 13. Hierbei sind drei Therapiestrahlen 13 unter einem Winkel 15 von zirka 45° ausgelenkt und ein Therapiestrahl 13 unter einem Winkel 15 von 0°. Am Ende 17 eines jeweiligen Therapiestrahls 13 befindet sich ein Behandlungsraum 19, der einen Behandlungsort 21 aufweist. Der Behandlungsort 21 bezeichnet den Ort, an dem der Therapiestrahl 13 mit dem hier nicht dargestellten Patienten wechselwirkt. Dieser Patient liegt, wie hier ebenfalls nicht dargestellt, auf einer Patientenliege. Der Behandlungsraum 19 ist von einer hier lediglich durch einen Kreis angedeuteten Abschirmung 23 umgeben.

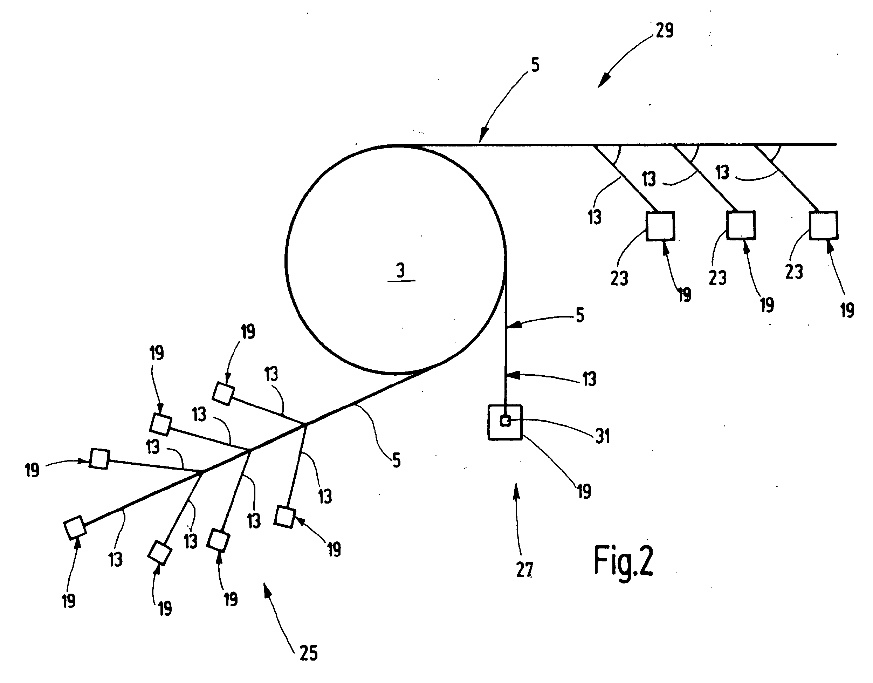

In Figur 2 ist die Strahlungsquelle 3 mit an verschiedenen Stellen ausgelenkten Hochenergiestrahlen 5 dargestellt. Die Behandlungsräume 19 sind hier an verschiedenen Orten an den Hochenergiestrahlen 5 angeordnet. Die Anordnung links im Bild weist eine Anordnung mit rechts und links vom Hochenergiestrahl 5 angeordneten Therapiestrahlen 13 und an deren Ende 17 angeordneten Behandlungsräumen 19 auf. Diese Anordnung ist mit der Bezugsziffer 25 gekennzeichnet. Mit der Bezugsziffer 27 ist eine Anordnung eines Behandlungsraumes 19 an einen Therapiestrahl 13 bezeichnet, der aus dem Hochenergiestrahl 5 unter 0° ausgelenkt ist. Diese Anordnung 27 kann einen Behandlungsraum 19 aufweisen, der eine größere räumliche Ausdehnung aufweist als die anderen Behandlungsräume 19. Dies wird dadurch möglich, dass durch die singuläre Anordnung eines einzigen Behandlungsraumes an dem Hochenergiestrahl ein größerer Platz verfügbar ist. Ein größerer Behandlungsraum19 kann beispielsweise dann notwendig sein, wenn zur Bestrahlung des Patienten eine mit der Bezugsziffer 31 bezeichnete Gantry notwendig ist, beziehungsweise in den Behandlungsraum eingebaut werden soll. Eine Gantry erlaubt die Rotation des Ionenstrahls um den Patienten. Auf die grundsätzlich bekannte Gantry 31 soll nicht näher eingegangen werden. Wichtig bleibt nur festzuhalten, dass es sich um eine Anordnung handelt, die den Therapiestrahl aus verschiedenen Richtungen auf den Patientenort lenkt. Hierbei handelt es sich um eine mehrere Tonnen schwere Anordnung.

In der mit 29 bezeichneten Anordnung ist gezeigt, dass aus einem Hochenergiestrahl 5 Therapiestrahlen 13 an einer Seite des Hochenergiestrahls angeordnet sind. Die Auslenkung erfolgt jeweils unter dem gleichen Winkel 15 sodass die Behandlungsräume 19 im Wesentlichen parallel nebeneinander in einer Reihe angeordnet sind. Es ist sowohl möglich, diese Therapiestrahlen 13 in einer Ebene von dem Hochenergiestrahl auszulenken, als auch derart auszulenken, dass die Behandlungsräume 19 nebeneinander liegend angeordnet sind, wobei eine Reihe in einem Stockwerk und die andere Reihe von Behandlungsräumen 19 in einem darüber- oder darunter liegenden Stockwerk, also in einer anderen Ebene, angeordnet ist.

Aus Figur 2 wird deutlich, dass die Anordnung von Behandlungsräumen 19, in die jeweils ein Therapiestrahl 13 eingeleitet wird, eine große Flexibilität im Aufbau von Behandlungsräumen in einer Bestrahlungseinrichtung erlaubt. Besonders ermöglicht wird diese Flexibilität dadurch, dass der Therapiestrahl jeweils von einem Hochenergiestrahl 5 unter einem Winkel 15 ausgelenkt wird. Wird als Strahlungsquelle 3 eine gepulste Strahlungsquelle ausgelegt, so kann die Abfolge der Bestrahlung der einzelnen Patienten sogar derart optimiert werden, dass in verschiedenen Behandlungsräumen gleichzeitig mehrere Patienten behandelt werden können, und der Hochenergiestrahl pulsweise in die verschiedenen Behandlungsräume 19 verteilt wird. Die um die Behandlungsräume 19 angeordnete Abschirmung 23 und die der Abschirmung dienenden Wände der Behandlungsräume erlauben es, dass sich in den Behandlungsräumen Patienten aufhalten können, ohne dass sie durch Neutronenstrahlung aus benachbarten Behandlungsräumen unnötig belastet und gefährdet werden.

An dieser Stelle sei auch noch einmal betont, dass die Behandlungsräume 19 nicht identisch sein müssen. Es ist vorgesehen, symmetrisch aufgebaute Behandlungsräume 19 und asymmetrisch aufgebaute Behandlungsräume 19 zu verwenden.

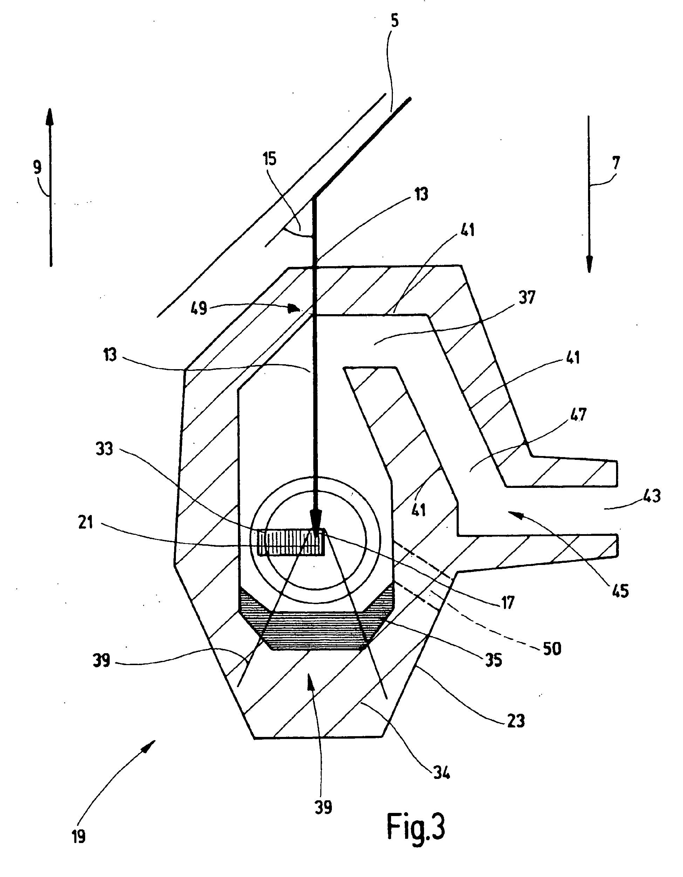

In Figur 3 ist ein Behandlungsraum 19, in den ein Therapiestrahl 13 eingeleitet wird gezeigt. Gleiche Teile sind mit gleichen Bezugsziffern versehen, sodass insofern auf die Beschreibung der vorigen Figuren verwiesen wird.

Es ist ein Teil des Hauptenergiestrahls 5, von dem der Therapiestrahl 13 unter einem Winkel 15 ausgeleitet wird, zu sehen. Am Ende 17 des Therapiestrahls 13 ist der Behandlungsort 21 zu sehen. Der Behandlungsort 21 weist eine Behandlungsliege 33 auf, auf die der Patient, der hier nicht dargestellt ist, gelegt wird und während der gesamten Bestrahlung verbleibt. Ebenfalls ist die Abschirmung 23 zu sehen. Die Abschirmung 23 weist Wände aus einem wasserstoffhaltigen Material, vorzugsweise Beton und/oder Gips und/oder Paraffinblöcke auf. Es ist zu erkennen, dass in Richtung des Pfeils 7, also strahlabwärts gesehen, die Abschirmung geschlossen ist. Ferner ist zu erkennen, dass neben dem Abschirmmaterial der Wände 34 strahlabwärts zusätzliches Abschirmmaterial vorgesehen ist. Dieses weist Metall auf, vorzugsweise Eisen oder Blei. Es ist ebenfalls möglich, außer Beton, ein anderes wasserhaltiges Material, insbesondere Gips, zu verwenden. Der Anteil des Wassers in diesem Material beträgt vorzugsweise zwischen 5 und 20 Gewichtsprozent. Die in dem Wasser enthaltenen Wasserstoffkerne, also die Protonen, moderieren die Neutronen aufgrund der fast identischen Masse und des damit verbundenen maximalen Impulsübertrags ideal. Gips, vorzugsweise abgebundener oder ausgehärteter Gips, der chemisch CaSO₄*2H₂O aufweist, ist besonders geeignet, Strahlung zu absorbieren. Das in Gips gebundene Wasser, auch als Kristallwasser bezeichnet, mit einem Gewichtsanteil von 5 bis 20%, stellt wie im Beton die Protonen zur Verfügung. Dadurch, dass Gips Calcium (Ca) mit einer Kernladungszahl von 20 enthält, werden Neutronen aber auch relativ wirksam Gammastrahlung abgeschirmt.

Im Gegensatz zu Gips weist regulärer Beton zusätzlich zu dem gebundenen Wasser auch kleinere Mengen an Metallen wie Calcium, Aluminium, Eisen oder Barium sowie Silicium auf. Diese Verunreinigungen im Beton können zu unerwünschten Kernreaktionen und zur Erzeugung von zusätzlicher Sekundärstrahlung führen. Des Weiteren weist Gips eine geringere Dichte von 2,1 g/cm³ auf als regulärer Beton, dessen Dichte zwischen ungefähr 2,8 g/cm³ beträgt. Somit ist Gips bei gleicher Abschirmwirkung leichter als Beton.

Vorzugsweise ist vorgesehen, diese Abschirmanordnung entweder aus Beton oder aus einer mehrschichtigen Anordnung von Beton und Gips aufzubauen. So ist es beispielsweise denkbar, eine Verschalung aus Beton, die relativ einfach herzustellen ist, aufzubauen und die zwischen den Verschalungswänden auftretenden Freiräume mit Gips aufzufüllen. Werden beide Materialien in Kombination verwendet, ist darauf zu achten, dass die Stabilität und die Abschirmwirkung optimiert werden.

Ferner ist vorgesehen, in die Abschirmung eine Neutronen-Absorberschicht einzufügen. Diese ist strahlabwärts des Bereiches, an dem Neutronenstrahlung entsteht, anzuordnen. Eine Neutronenabsorberschicht enthält vorzugsweise Bor, bor-haltiges Glas und/oder Bor-Paraffin. Ferner kann auch Cadmium oder Gadolinium verwendet werden.

Die Abschirmung ist in ihrer einfachsten Ausgestaltung G-förmig ausgelegt, wobei der Behandlungsort sich innerhalb der G-förmig angeordneten Wände befindet. In einer weiteren Ausgestaltung ist es ebenfalls denkbar, die Anordnung der Wände 34 spiralförmig vorzusehen, sodass der Behadlungsort durch mehrere Wände 34 abgeschirmt ist.

Die Abschirmung 23 weist einen Zugang 37 auf. Dieser Zugang ist strahlaufwärts, also in Richtung des Pfeils 9 vom Behandlungsort 21 her gesehen, angeordnet. Aus Figur 3 wird deutlich, dass der Zugang 37 im Bereich der Eintrittsstelle des Therapiestrahls 13 in den Behandlungsraum 19 angeordnet ist, also auf der Seite des Behandlungsraums 19, auf der der Therapiestrahl 13 in diesen eintritt.

Sollten im Bereich des Therapiestrahls 13 innerhalb des Behandlungsraums 19 Umlenkeinrichtungen und/oder eine Gantry vorgesehen werden, so ist bei der hier vorgesehenen Anordnung des Zugangs 37 sichergestellt, dass auch bei Ausfall derartiger Umlenkeinrichtungen der Zugang 37 nicht unmittelbar mit dem Therapiestrahl 13 oder mit Sekundärstrahlen beaufschlagt wird.

Die Anordnung des Zugangs 37 auf der Seite der Eintrittsstelle des Therapiestrahls 13 in den Behandlungsraum 19 bietet besonderen Schutz vor Neutronenstrahlung. Entsteht also an dem Behandlungsort 21 durch die Wechselwirkung der hochenergetischen Ionen des Therapiestrahls 13 mit dem Gewebe eines hier nicht dargestellten Patienten Sekundärstrahlung, insbesondere Neutronenstrahlung, so ist der Zugang 37 davon nicht direkt betroffen. Der Kegel der Neutronenstrahlung ist hier mit der Bezugsziffer 39 angedeutet.

Die Neutronenstrahlung verläuft im Übrigen in Richtung des Pfeils 7, also strahlabwärts, so dass, wie gesagt, der Zugang 37 aufgrund der gewählten Anordnung davon nicht unmittelbar betroffen sein kann, allenfalls von Reflexionen der Sekundär- beziehungsweise Neutronenstrahlung.

Die Abschirmung des Neutronenkegels 39 von Bereichen außerhalb des Behandlungsorts 21 erfolgt durch die Wand 34 sowie durch die Metall aufweisende Abschirmung 35. An diesem strahlabwärts gerichteten Ende des Behandlungsortes 19 ist die Wand 34 dicker ausgelegt als an den Seitenwänden der Abschirmung. Der Zugang selbst ist labyrinthartig ausgelegt. Dies hat den Vorteil, dass rückgestreute Neutronen, also Neutronen, die sich in Richtung des Pfeils 9 bewegen, durch Streuung an den Innenflächen 41 der Wände 34 zusätzlich abgeschwächt werden.

Es bleibt festzuhalten, dass die Abschirmung durch mehrere Effekte erfolgt: Die vorwärts gerichtete, im Bereich des Kegels 39 vorhandene Neutronenstrahlung wird durch die Abschirmung 34 und 35, also durch die Wände des Behandlungsraumes 19 absorbiert. Neutronen, die durch den Zugang 37 aus dem Behandlungsraum austreten könnten, werden durch Reflektion an den Innenflächen der Wände 34 in ihrer Intensität abgeschwächt. Somit ist sichergestellt, dass an den Ausgang 43 des Zugangslabyrinths 45 keine Neutronen gelangen.

Eine Person, die in den Behandlungsraum gelangen will, betritt das Zugangslabyrinth 45 an dem Ausgang 43 und bewegt sich entlang des Ganges 47, um zu dem Zugang 37 zu gelangen. Von diesem Zugang 37 gelangt die Person ohne Weiteres in den Behandlungsraum und somit an den Behandlungsort. Die zu bestrahlenden Patienten werden ebenfalls durch das Zugangslabyrinth 45 entlang des Weges 47 in den Behandlungsraum befördert. Der Therapiestrahl 13 tritt über ein hier nicht dargestelltes Fenster durch die dem Behandlungsort gegenüberliegende Wand 49 in den Behandlungsraum.

In Figur 3 ist noch eine als Not- und/oder Servicezugang dienende Tür 50 gestrichelt eingezeichnet, die ebenso wie die Abschirmung 23 gegen einen Durchtritt der entstehenden Strahlung geschützt ist. Wegen des hohen Gewichts kann die Tür auf geeignete Weise bewegbar, insbesondere versenkbar sein, beispielsweise durch einen elektrischen, pneumatischen und/oder hydraulischen Antrieb.

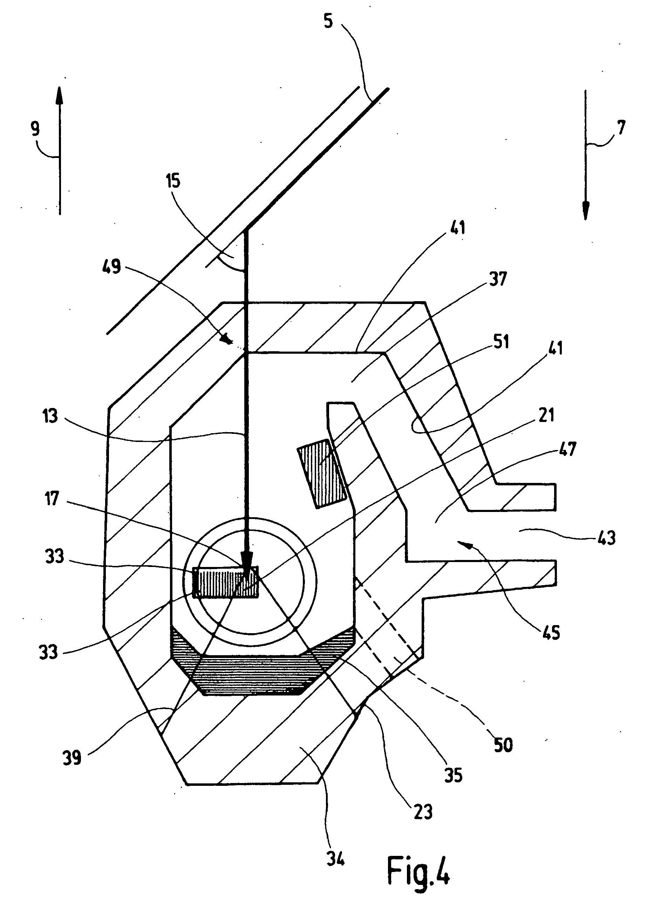

Figur 4 zeigt ebenfalls einen Behandlungsraum 19, für den das zu Figur 3 Gesagte gilt. Gleiche Teile sind mit gleichen Bezugsziffern versehen, sodass insofern auf die Beschreibung der vorigen Figuren verwiesen wird. Der Unterschied ist der, dass dieser Behandlungsraum im Gegensatz zu dem in Figur 3 dargestellten asymmetrisch ausgelegt ist. Aus diesem Grunde ist in Figur 4 eine zusätzliche Behandlungsliege, auf die der Patient liegt, gezeigt. Dies bietet den Vorteil, die Zeiten zwischen den Betrahlungen zu verkürzen. Der bereits bestrahlte Patient befindet sich auf der Behandlungsliege 33 und der zu bestrahlende Patient wird auf der Liege 51 hereingeschoben. Somit ist ein rascher Patientenwechsel möglich.

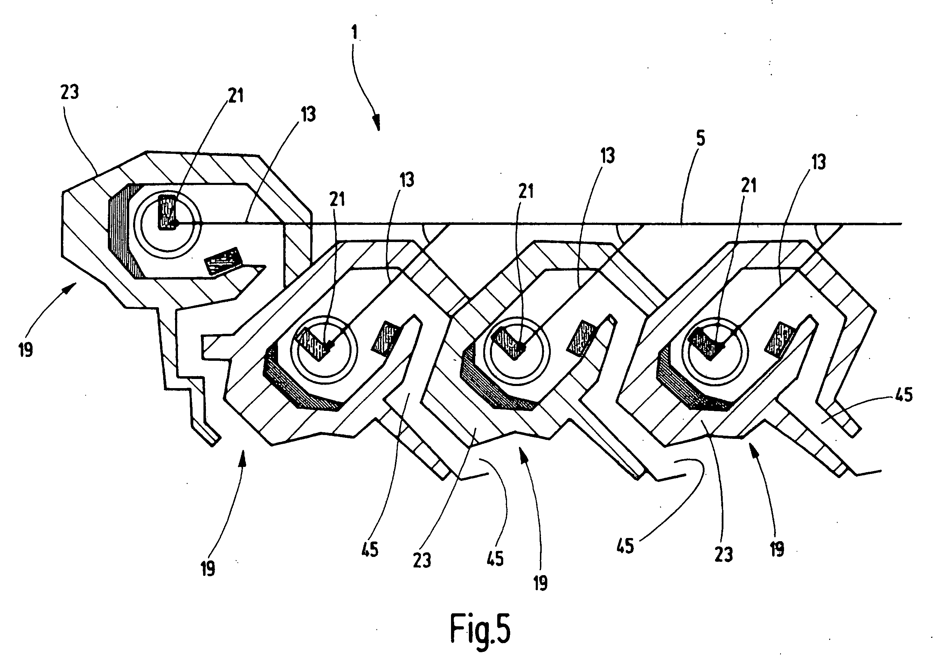

In Figur 5 ist eine Anordnung von vier Behandlungsräumen 19 gezeigt. Gleiche Teile sind mit gleichen Bezugsziffern versehen, sodass insofern auf die Beschreibung der vorigen Figuren verwiesen wird. Der Therapiestrahl 13 wird hier unter 45° aus dem Hochenergiestrahl 5 ausgelenkt. Unter 0°, das heißt in Richtung des Hochenergiestrahls 5, liegt ein Behandlungsplatz, bei dem der Therapiestrahl 13 ohne Auslenkung aus dem Hauptstrahl 5 gewonnen wird. Gezeigt sind der Behandlungsort 21 sowie die Abschirmung 33 mit dem Zugangslabyrinth 45. Zu erkennen ist an dieser Anordnung, dass die Wände 34 zur Abschirmung 23 des jeweiligen Nachbarbehandlungsraumes 19 verwendet werden. Dies spart Kosten und erlaubt eine relativ räumlich dichte Anordnung der Behandlungsräume 19 um den Hochenergiestrahl 5.

Vorzugsweise ist eine solche Anordnung von nebeneinander in einer Reihe angeordneten Bestrahlungsräumen 19 auch in mindestens zwei Ebenen eines Gebäudes vorgesehen. Hierzu wird der Strahl nicht nur in einer Ebene ausgelenkt, sondern unter einem Winkel zu dieser Ebene und wird vorzugsweise von oben in einen Behandlungsraum eingeleitet, und dort beispielsweise zu einem horizontalen Strahl umgelenkt oder in eine Gantry 31 eingelenkt, die die Bestrahlung eines Patienten unter verschiedenen Winkeln erlaubt.

Insgesamt wird deutlich, dass mindestens zwei, vorzugsweise eine Anzahl von Behandlungsräumen praktisch parallel zueinander - quasi in einem Fischgrätmuster - sehr raumsparend angeordnet werden können, ohne dass die Zugänge zu diesen beeinträchtigt würden. Es ist möglich, Zugangslabyrinthe zu realisieren und dazu die Außenwände von benachbarten Behandlungsräumen zu nutzen. Dadurch kann die Bestrahlungseinrichtung ohne irgendwelche Sicherheitseinbußen sehr kompakt aufgebaut sein. Die Nutzung der Bestrahlungseinrichtung ist deshalb sehr intensiv und kostengünstig, weil den einzelnen Behandlungsräumen separate, getrennt steuerbare, also auch ein- und ausschaltbare, Therapiestrahlen zugeführt werden. Da die Behandlungsräume optimal gegeneinander abgeschirmt sind, kann ein Behandlungsraum von Bedienungspersonal gefahrlos betreten werden, auch wenn in benachbarten Behandlungsräumen Bestrahlungen durchgeführt werden.

## Patentansprüche

1. Bestrahlungseinrichtung für die Protonen- und/oder Ionenstrahltherapie mit einer Strahlungsquelle (3), mit mindestens zwei eine Abschirmung (23) und einen Behandlungsort (21) aufweisenden Behandlungsräumen (19), in die ein Therapiestrahl (13) einleit bar ist, wobei in jeden der Behandlungsräume (19) ein separater Therapiestrahl (13) ein bar ist, und mit mindestens einem in den jeweiligen Behandlungsraum führenden Zugang (37), **dadurch gekennzeichnet, dass** der Zugang, in Richtung des Therapiestrahls (13) gesehen, strahlaufwärts zum Behandlungsart (21), nämlich im Bereich der Eintrittsstelle des Therapiestrahls (13), in den Behandlungsraum (19) angeordnet ist.

2. Bestrahlungseinrichtung nach Anspruch 1, **dadurch gekennzeichnet, dass** der mindestens eine Behandlungsraum (19) symmetrisch oder asymmetrisch ist.

3. Bestrahlungseinrichtung nach Anspruch 1 oder 2, **dadurch gekennzeichnet, dass** die Behandlungsräume (19) -in Strahlrichtung des Therapiestrahls (13) gesehen- durch die Abschirmung (23) abgeschlossen sind.

4. Bestrahlungseinrichtung nach einem der vorhergehenden Ansprüche, **dadurch gekennzeichnet, dass** der Zugang (37) ein Labyrinth aufweist.

5. Bestrahlungseinrichtung nach Anspruch 4, **dadurch gekennzeichnet, dass** das Labyrinth durch separate Wände realisiert wird.

6. Bestrahlungseinrichtung nach einem der Ansprüche 4 oder 5, **dadurch gekennzeichnet, dass** das Labyrinth durch Wände benachbarter Behandlungsräume (19) realisiert wird.

7. Bestrahlungseinrichtung nach einem der vorhergehenden Ansprüche, **dadurch gekennzeichnet, dass** der Zugang teilweise entlang einer Außenwand des jeweiligen Behandlungsraumes verläuft.

8. Bestrahlungseinrichtung nach einem der vorhergehenden Ansprüche, **dadurch gekennzeichnet, dass** der Therapiestrahl (13) aus einem aus der Strahlungsquelle (3) austretenden Hochenergiestrahl (5) ausgelenkt wird.

9. Bestrahlungseinrichtung nach einem der vorhergehenden Ansprüche, **dadurch gekennzeichnet, dass** die Auslenkung des Therapiestrahls unter einem Winkel α erfolgt.

10. Bestrahlungseinrichtung nach einem der vorhergehenden Ansprüche, **dadurch gekennzeichnet, dass** der Winkel α 0 ° bis 90° beträgt.

11. Bestrahlungseinrichtung nach einem der vorhergehenden Ansprüche, **dadurch gekennzeichnet, dass** die Behandlungsräume (19) in Reihe -vorzugsweise parallel- nebeneinander liegen.

12. Bestrahlungseinrichtung nach einem der vorhergehenden Ansprüche, **dadurch gekennzeichnet, dass** die Behandlungsräume (19) in zwei Reihen in einer Ebene -vorzugsweise parallelnebeneinander liegen.

13. Bestrahlungseinrichtung nach einem der vorhergehenden Ansprüche, **dadurch gekennzeichnet, dass** die Behandlungsräume (19) in unterschiedlichen Ebenen liegen.

14. Bestrahlungseinrichtung nach einem der vorhergehenden Ansprüche, **dadurch gekennzeichnet, dass** die Behandlungsräume (19) sternförmig um die Strahlungsquelle (3) angeordnet sind.

15. Bestrahlungseinrichtung nach einem der vorhergehenden Ansprüche, **dadurch gekennzeichnet, dass** die Abschirmung (23) eines Behandlungsraumes (19) Metall und/oder ein wasserstoffhaltiges Material und/oder Graphit aufweist.

16. Bestrahlungseinrichtung nach Anspruch 15, **dadurch gekennzeichnet, dass** das wasserstoffhaltige Material Paraffin und/oder gebundenes Wasser aufweist.

17. Bestrahlungseinrichtung nach Anspruch 16, **dadurch gekennzeichnet, dass** das Material, welches gebundenes Wasser aufweist, Beton und/oder Gips und/oder eine Kombination daraus ist.

18. Bestrahlungseinrichtung nach Anspruch 15, **dadurch gekennzeichnet, dass** das Metall Eisen und/oder Blei umfasst.

19. Bestrahlungseinrichtung nach einem der vorhergehenden Ansprüche, **dadurch gekennzeichnet, dass** die Abschirmung ein Neutronen absorbierendes Material aufweist.

20. Bestrahlungseinrichtung nach Anspruch 19, **dadurch gekennzeichnet, dass** das Neutronen absorbierende Material Bor und/oder Bor-Paraffin und/oder Cadmium und/oder Gadolinium ist.

21. Bestrahlungseinrichtung nach einem der vorhergehenden Ansprüche, **dadurch gekennzeichnet, dass** die Wände der Behandlungsräume G-förmig angeordnet sind.

22. Bestrahlungseinrichtung nach einem der vorhergehenden Ansprüche, **dadurch gekennzeichnet, dass** die Wände der Behandlungsräume spiralförmig um den Behandlungsort angeordnet sind.

23. Bestrahlungseinrichtung nach einem der vorhergehenden Ansprüche, **dadurch gekennzeichnet, dass** die Behandlungsräume eine als Not- und/oder Servicezugang dienende Tür aufweisen.

24. Bestrahlungseinrichtung nach Anspruch 23, **dadurch gekennzeichnet, dass** die Tür Material der Abschirmung umfasst.

25. Bestrahlungseinrichtung nach Anspruch 23, **dadurch gekennzeichnet, dass** die Tür ein Teil der Abschirmung ist.

26. Bestrahlungseinrichtung nach Anspruch 23, **dadurch gekennzeichnet, dass** die Tür versenkbar ist.

## Claims

1. An irradiation facility for proton and/or ion beam therapy having a radiation source (3), having at least two treatment rooms (19) comprising a shielding (23) and a treatment place (21), into which a therapy beam (13) can be guided, whereby a separate therapy beam (13) can be guided into each of the treatment rooms (19), and having at least one access (37) leading to the corresponding treatment room, **characterized in that** the access, as seen in the direction of the therapy beam (13), is arranged up-beam with respect to the treatment place (21) namely in the area of the site of entry of the therapy beam (13) into the treatment room (19).

2. An irradiation facility according to claim 1, **characterized in that** the at least one treatment room (19) is symmetrical or asymmetrical.

3. An irradiation facility according to claim 1 or 2, **characterized in that** the treatment rooms (19) - as seen in the beam direction of the therapy beam (13) - are closed off by the shielding (23).

4. An irradiation facility according to any one of the preceding claims, **characterized in that** the access (37) comprises a labyrinth.

5. An irradiation facility according to claim 4, **characterized in that** the labyrinth is realized by means of separate walls.

6. An irradiation facility according to any one of the claims 4 or 5, **characterized in that** the labyrinth is realized by walls of neighboring treatment rooms (19).

7. An irradiation facility according to any one of the preceding claims, **characterized in that** part of the access extends along an external wall of the corresponding treatment room.

8. An irradiation facility according to any one of the preceding claims, **characterized in that** the therapy beam (13) is deflected from a high energy beam (5) emanating from the radiation source (3).

9. An irradiation facility according to any one of the preceding claims, **characterized in that** the therapy beam is deflected at an angle α.

10. An irradiation facility according to any one of the preceding claims, **characterized in that** the angle α is 0° to 90°.

11. An irradiation facility according to any one of the preceding claims, **characterized in that** the treatment rooms (19) are situated in series - preferably parallel - next to each other.

12. An irradiation facility according to any one of the preceding claims, **characterized in that** the treatment rooms (19) are situated in two rows in one plane - preferably parallel - next to each other.

13. An irradiation facility according to any one of the preceding claims, **characterized in that** the treatment rooms (19) are situated in different planes.

14. An irradiation facility according to any one of the preceding claims, **characterized in that** the treatment rooms (19) are arranged in the shape of a star around the radiation source (3).

15. An irradiation facility according to any one of the preceding claims, **characterized in that** the shielding (23) of a treatment room (19) comprises metal and/or a hydrogen-containing material and/or graphite.

16. An irradiation facility according to claim 15, **characterized in that** the hydrogen-containing material comprises paraffin and/or bound water.

17. An irradiation facility according to claim 16, **characterized in that** the material comprising bound water is concrete and/or gypsum and/or a combination thereof.

18. An irradiation facility according to claim 15, **characterized in that** the metal comprises iron and/or lead.

19. An irradiation facility according to any one of the preceding claims, **characterized in that** the shielding comprises a neutron-absorbing material.

20. An irradiation facility according to claim 19, **characterized in that** the neutron-absorbing material is boron and/or boron-paraffin and/or cadmium and/or gadolinium.

21. An irradiation facility according to any one of the preceding claims, **characterized in that** the walls of the treatment rooms are arranged in a G-shaped arrangement.

22. An irradiation facility according to any one of the preceding claims, **characterized in that** the walls of the treatment rooms are arranged in a spiral arrangement around the treatment place.

23. An irradiation facility according to any one of the preceding claims, **characterized in that** the treatment rooms comprise a door that serves as emergency and/or service access.

24. An irradiation facility according to claim 23, **characterized in that** the door comprises material of the shielding.

25. An irradiation facility according to claim 23, **characterized in that** the door is a part of the shielding.

26. An irradiation facility according to claim 23, **characterized in that** the door is retractable.

## Revendications

1. Dispositif d'irradiation pour la protonthérapie et/ou l'ionothérapie avec une source d'irradiation (3), avec au moins deux salles de traitement (19) présentant un blindage (23) et un site de traitement (21) dans lesquelles un rayon thérapeutique (13) peut être introduit, un rayon thérapeutique séparé (13) pouvant être introduit dans chacune des salles de traitement (19), et avec au moins un accès (37) menant dans la salle de traitement respective, **caractérisé en ce que l'**accès est disposé, vu en direction du rayon thérapeutique (13), en amont du rayon vers le site de traitement (21), à savoir dans la zone du point d'entrée du rayon thérapeutique (13), dans la salle de traitement (19).

2. Dispositif d'irradiation selon la revendication 1, **caractérisé en ce que** l'au moins une salle de traitement (19) est symétrique ou asymétrique.

3. Dispositif d'irradiation selon la revendication 1 ou 2, **caractérisé en ce que** les salles de traitement (19) sont fermées par le blindage (23), vu en direction du rayon thérapeutique (13).

4. Dispositif d'irradiation selon l'une quelconque des revendications précédentes, **caractérisé en ce que** l'accès (37) présente un labyrinthe.

5. Dispositif d'irradiation selon la revendication 4, **caractérisé en ce que** le labyrinthe est réalisé par des parois séparées.

6. Dispositif d'irradiation selon l'une quelconque des revendications 4 ou 5, **caractérisé en ce que** le labyrinthe est réalisé par des parois de salles de traitement voisines (19).

7. Dispositif d'irradiation selon l'une quelconque des revendications précédentes, **caractérisé en ce que** l'accès s'étend partiellement le long d'une paroi externe de la salle de traitement respective.

8. Dispositif d'irradiation selon l'une quelconque des revendications précédentes, **caractérisé en ce que** le rayon thérapeutique (13) est dévié d'un rayon à haute énergie (5) sortant de la source d'irradiation (3).

9. Dispositif d'irradiation selon l'une quelconque des revendications précédentes, **caractérisé en ce que** la déviation du rayon thérapeutique s'effectue avec un angle α.

10. Dispositif d'irradiation selon l'une quelconque des revendications précédentes, **caractérisé en ce que** l'angle α est de 0° à 90°.

11. Dispositif d'irradiation selon l'une quelconque des revendications précédentes, **caractérisé en ce que** les salles de traitement (19) se situent en rangée, de préférence parallèlement, l'une à côté de l'autre.

12. Dispositif d'irradiation selon l'une quelconque des revendications précédentes, **caractérisé en ce que** les salles de traitement (19) se situent en deux rangées dans un plan, de préférence parallèlement, l'une à côté de l'autre.

13. Dispositif d'irradiation selon l'une quelconque des revendications précédentes, **caractérisé en ce que** les salles de traitement (19) se situent dans des plans différents.

14. Dispositif d'irradiation selon l'une quelconque des revendications précédentes, **caractérisé en ce que** les salles de traitement (19) sont disposées en forme d'étoile autour de la source d'irradiation (3).

15. Dispositif d'irradiation selon l'une quelconque des revendications précédentes, **caractérisé en ce que** le blindage (23) d'une salle de traitement (19) présente du métal et/ou une matière contenant de l'hydrogène et/ou du graphite.

16. Dispositif d'irradiation selon la revendication 15, **caractérisé en ce que** la matière contenant de l'hydrogène présente de la paraffine et/ou de l'eau combinée.

17. Dispositif d'irradiation selon la revendication 16, **caractérisé en ce que** la matière qui présente de l'eau combinée est du béton et/ou du plâtre et/ou une combinaison de ceux-ci.

18. Dispositif d'irradiation selon la revendication 15, **caractérisé en ce que** le métal comprend du fer et/ou du plomb.

19. Dispositif d'irradiation selon l'une quelconque des revendications précédentes, **caractérisé en ce que** le blindage présente une matière absorbant les neutrons.

20. Dispositif d'irradiation selon la revendication 19, **caractérisé en ce que** la matière absorbant les neutrons est du bore et/ou du bore-paraffine et/ou du cadmium et/ou du gadolinium.

21. Dispositif d'irradiation selon l'une quelconque des revendications précédentes, **caractérisé en ce que** les parois des salles de traitement sont disposées en forme de G.

22. Dispositif d'irradiation selon l'une quelconque des revendications précédentes, **caractérisé en ce que** les parois des salles de traitement sont disposées en forme de spirale autour du site de traitement.

23. Dispositif d'irradiation selon l'une quelconque des revendications précédentes, **caractérisé en ce que** les salles de traitement présentent une porte servant d'accès d'urgence et/ou de service.

24. Dispositif d'irradiation selon la revendication 23, **caractérisé en ce que** la porte comprend de la matière du blindage.

25. Dispositif d'irradiation selon la revendication 23, **caractérisé en ce que** la porte fait partie du blindage.

26. Dispositif d'irradiation selon la revendication 23, **caractérisé en ce que** la porte peut être abaissée.
